(19) **Europäisches Patentamt**
**European Patent Office**
**Office européen des brevets**

(11) **EP 2 240 591 B2**

(12) **NEW EUROPEAN PATENT SPECIFICATION**
After opposition procedure

(45) Date of publication and mention
of the opposition decision:
**05.04.2023 Bulletin 2023/14**

(45) Mention of the grant of the patent:
**14.11.2012 Bulletin 2012/46**

(21) Application number: **09710344.4**

(22) Date of filing: **10.02.2009**

(51) International Patent Classification (IPC):
*C12P 7/06* (2006.01)    *C12P 19/00* (2006.01)
*C12M 1/36* (2006.01)

(52) Cooperative Patent Classification (CPC):
**C12P 19/00; C12P 7/06;** Y02E 50/10

(86) International application number:
**PCT/SE2009/000078**

(87) International publication number:
**WO 2009/102256 (20.08.2009 Gazette 2009/34)**

(54) **METHOD OF PRODUCTION OF ETHANOL FROM TWO DIFFERENT STARTING MATERIALS**

VERFAHREN ZUR HERSTELLUNG VON ETHANOL AUS ZWEI VERSCHIEDENEN STARTMATERIALIEN

PROCÉDÉ DE PRODUCTION D'ÉTHANOL À PARTIR DE DEUX MATÉRIAUX DIFFÉRENTS DE DÉPART

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **11.02.2008 SE 0800304**

(43) Date of publication of application:
**20.10.2010 Bulletin 2010/42**

(60) Divisional application:
**12192280.1 / 2 562 262**

(73) Proprietor: **Sekab E-Technology AB**
**891 24 Örnsköldsvik (SE)**

(72) Inventors:
• **ÖHGREN GREDEGÅRD, Karin**
**S-224 66 Lund (SE)**
• **ZACCHI, Guido**
**S-214 58 Malmö (SE)**

(74) Representative: **Kransell & Wennborg KB**
**P.O. Box 27834**
**115 93 Stockholm (SE)**

(56) References cited:
• **ÖHGREN ET AL: "Simultaneous saccharification and co-fermentation of glucose and xylose in stem-pretreated corn stover at high fiber content with Saccharomyces cerevisiae TMB3400" JOURNAL OF BIOTECHNOLOGY, vol. 126, 2006, pages 488-498, XP024956581**
• **LARSSON ET AL: "The generation of fermentation inhibitors during dilute acid hydrolysis of softwood" ENZYME AND MICROBIAL TECHNOLOGY, vol. 24, 1999, pages 151-159, XP003024061**
• **SZENGYEL ET AL: "Effect of acetic acid and furfural on cellulase production of Trichoderma reesei RUT C30" APPLIED BIOCHEMISTRY AND BIOTECHNOLOGY, vol. 89, 2000, pages 31-42, XP002537086**
• **SASSNER ET AL: "Techno-economic evaluation of bioethanol production from three different lignocellulosic materials" BIOMASS AND BIOENERGY, vol. 32, 11 December 2007 (2007-12-11), pages 422-430, XP022664170**
• **HAHN-HÄGERDAL ET AL: "Bio-ethanol - the fuel of tomorrow from the residues of today" TRENDS IN BIOTECHNOLOGY, vol. 24, 16 October 2006 (2006-10-16), pages 549-556, XP025052220**
• **LINDE ET AL: "Bioethanol production from non-starch carbohydrate residues in process streams from a dry-mill ethanol plant" BIORESOURSE TECHNOLOGY, vol. 99, 20 February 2008 (2008-02-20), pages 6505-6511, XP022679226**

EP 2 240 591 B2

## Description

### Field of invention

The present disclosure relates to ethanol production using at least two starting materials; one which contains lignocellulosic material and another which is high in fermentable sugars, starch or another material which easily can be converted to fermentable sugars. Accordingly, the production of ethanol may be based on biomass as a whole, such as different crops as a whole. Referring to for example the corn plant, the fermentable sugars may be obtained from the corn cob as well as from the corn stover. Other examples are different types of grain and the straw, respectively, and, regarding sugar canes, the extracted juice (containing fermentable sugars) and the bagass, respectively. Other crops than those listed can also be used as raw material. Of course, the raw material can also be composed of a mixture of the above-mentioned plants and any other suitable crop or part of crop.

### State of the art

A literature study named "Feasibility Study for Co-locating and integrating Ethanol Production Plants from Corn Starch and Lignocellulosic Feedstocks", published by the U.S. Department of Agriculture and U.S. Department of Energy (Revised January 2005) relates to the above-mentioned field. The study addresses the problem of identifying scenarios where capital equipment, operating expenses and co-products could be shared in order to find overall savings compared to a "stand alone" cellulosic facility using corn stover feedstock. Seven hypothetical scenarios are discussed, of which two (scenarios 6 and 7) involve two lines for processing of corn cobs and corn stover, respectively, to fermentable sugars and subsequent merge of the two saccharide flows. On page 19 of the report it is concluded that:

*"Scenarios 4-7 suffer from the loss of the DDGS-co-product value.*
*Scenarios 5a and b suffer from reduced ethanol production. Scenario 4 vs. 7 show there is no benefit to combining fermentations, probably because the tanks scale linearly. The only combined scenarios that have better economics than a stand alone stover facility are scenarios 2, 3 and 5b; combined utilities, ethanol purification and combined C6 fermentation with the C5 stream being sold separately, due to the economies of scale available in distillation equipment and the added value of the C5 steam in the last case.".*

### Description

As apparent from the above-mentioned publication, the person skilled in the art is advised not to combine the two saccharide flows in the fermentation step.

It is an object of some aspects of the present disclosure to provide for increased yields in ethanol production, in particular in processes using one starting material which contains lignocellulose and another which is high in fermentable sugars, starch or another material which easily can be converted to fermentable sugars.

It is another object of some aspects of the present disclosure to provide for energy efficient ethanol production, which in turn contributes to a minimization of the total production cost for ethanol.

Thus, as a first, non-claimed, configuration of a first, non-claimed, aspect of the present disclosure, there is provided a method of improving the ethanol yield in production of an ethanol-containing product from a lignocellulosic biomass and a sugar product containing fermentable sugars derived from a sugar-rich material, comprising:

treatment, involving hydrolysis, of said lignocellulosic biomass in one or more steps to obtain lignocellulose-derived treatment products including fermentable sugars and substancer capable of stressing the fermenting agent ; and fermentation, using a fermenting agent, of a mixture comprising at least part of said lignocellulose-derived treatment products and said fermentable sugars derived from said sugar-rich material to obtain the ethanol-containing product, wherein an amount of said sugar product is mixed with an amount of at least one of the following:

(i) lignocellulose-derived material in the treatment;
(ii) lignocellulose-derived treatment products from the treatment; and
(iii) lignocellulose-derived treatment products in the fermentation,

such that said fermentable sugars derived from said sugar-rich material and said at least part of said lignocellulose-derived treatment products are present in the mixture, and said amounts are controlled such that the fermenting agent is subjected to stress by lignocellulose-derived treatment products to the extent that the ethanol yield is improved.

In the context of the present disclosure, "lignocellulosic biomass" refers to organic material which contains cellulose, hemicellulose and lignin. The cellulose and hemicellulose are carbohydrate polymers which are tightly associated with the lignin in the lignocellulosic biomass. Examples of lignocellulosic biomass are wood and wood residues, municipal paper waste, agricultural residues such as corn stover, bagass and straw and dedicated energy crops such as woody grasses.

Further, "sugar-rich material" refers to a biomass which has a high content of fermentable sugars. Examples of sugar-rich materials are sugar cane, sugar beet and sweet sorghum. Thus, the "sugar product containing fermentable sugars derived from a sugar-rich material" may be an extract from sugar cane, sugar beet or sweet sorghum. Sometimes such an extract is referred to as a "juice" within the art. The sugar product may also be molasses, which may be provided by further processing of the above-mentioned extract. The processing may comprise concentration, which may be beneficial if the sugar product is to be stored before it is added in the method. Storage may be required because the sugar-rich material may normally not be harvested all year around. Also, the sugar product may comprise crystallized sugar.

"Fermentable sugars" refer to sugar molecules which may be fermented by a fermenting agent, such as a yeast or bacterium. Thus, which sugars to be considered "fermentable sugars" may depend on the employed fermenting agent. The yeast may for example be *saccharomyces cerevisiae* or genetically engineered variants thereof, and the bacterium may for example be a genetically engineered variant of *e. coli.* The fermentable sugars comprise e.g., monosaccharides, such as pentoses and hexoses, and disaccharides. The fermentable sugars of the cellulose-derived treatment products normally comprise pentoses and hexoses. The fermentable sugars derived from a sugar-rich material normally comprise hexose mono- and disaccharides (e.g., sucrose). The "treatment" may include various steps for the degradation of the lignocellulosic biomass. A hydrolysis is however always performed to obtain the lignocellulose-derived fermentable sugars. The hydrolysis refers to the process in which the majority of the polysaccharides are degraded to fermentable sugars such as monosaccharides. In addition to the lignocellulose-derived fermentable sugars, the lignocellulose-derived treatment products resulting from the "treatment" comprise substances capable of stressing the fermenting agent (herein also referred to as inhibitors). As an example, the "treating" may comprise a pretreatment, which may for example comprise application of heat and overpressure. In such a pretreatment, some of the inhibitors are formed and/or released. Further, the treatment may comprise impregnation of the lignocellulosic biomass, normally as the first step of the treatment, i.e., before any pretreatment or hydrolysis. The impregnation may comprise application of an impregnation fluid, which may be a liquid or a gas. The impregnation fluid may comprise water, acid and/or other chemicals. For example, the impregnation fluid may be $SO_2(aq)$ or $SO_2(g)$. The impregnation fluid may for example be applied by means of spraying.

Thus, in the context of the present disclosure, the "lignocellulose-derived treatment products" refers to the products obtained after hydrolysis and optionally one or more other treatments of the lignocellulosic biomass, wherein the purpose of the one or more other treatments also is to degrade, or facilitate degradation of, the lignocellulosic biomass. An example of such an "other treatment" is a pretreatment to disrupt the structure of the lignocellulosic biomass so as to render the cellulose in it accessible for the subsequent hydrolysis.

"Fermentation" refers to biological conversion of the fermentable sugars to ethanol. In the fermentation, a "fermenting agent" is employed. The fermenting agent of the present disclosure refers to an microorganism capable of converting monosaccharides and/or disaccharides to ethanol. The microorganism may be wild-type or genetically engineered. Further, the microorganism may be a mutant selected for having a certain property, or properties, beneficial for the ethanol production. The fermenting agent may thus be a yeast, such as *saccharomyces cerevisiae,* or an bacterium. The "fermenting agent" may also be a mixture of fermenting agents. The fermenting agent(s) may be capable of fermenting pentoses.

The mixture subjected to fermentation comprises at least part of said lignocellulose-derived hydrolysis products and said fermentable sugars derived from said sugar-rich material. Consequently, the mixture comprises fermentable sugars derived from two different starting materials. If the method comprises simultaneous saccharification and fermentation, the mixture will also comprise cellulosic polysaccharides which are degraded to fermentable sugars by enzymes.

The proportion of the different components of the mixture is determined in the mixing of the cellulose-derived material and the sugar product. Such a mixing may take place during any stage of the treatment, after the treatment or during the fermentation. Consequently, the sugar product may be mixed with the cellulose-derived material before or after the cellulose and hemicelluloses of the cellulose-derived material are degraded to fermentable sugars. By controlling the amounts in the mixing, the concentration of the lignocellulose-derived treatment products in the mixture is also controlled. A higher amount of the lignocellulose derived material provided in the mixing will typically result in a larger proportion of lignocellulose-derived treatment products in the mixture.

Some of the lignocellulose-derived treatment products stress the fermenting agent. If their concentrations are too high in the fermentation, the fermenting agent is inhibited and the ethanol yield is decreased. However, in lower concentrations, they stimulate the fermenting agent to produce more ethanol. Consequently, the extent of which the fermenting agent is stressed may be determined by controlling the amounts in the mixing.

In a pure lignocellulosic hydrolysate, i.e. the product of a hydrolysis of only a lignocellulosic biomass, the concentrations of the lignocellulose-derived treatment products in question are normally too high to achieve an optimal result. That is, an inhibiting effect is often obtained. In contrast, in the sugar product or the hydrolysate of a starch-rich biomass (see below), the concentrations of the lignocellulose-derived treatment products in question are too low to stimulate the fermenting agent to a substantial degree. Therefore, mixing of material derived from the two starting materials in controlled amounts will increase the ethanol yield. The ethanol yield refers to the total amount of ethanol obtained from the amounts of lignocellulose-derived material and sugar product, respectively, after fermentation.

Accordingly, the concentrations of the lignocellulose-derived treatment products in question correspond to the amount of provided lignocellulose biomass, but they also depend on the type of pretreatment and saccharification process applied. Therefore, the optimal amount of provided lignocellulose biomass may be determined for each given process. Using common general knowledge and the teachings of the present disclosure, the person skilled in the art may perform such an optimization without undue burden. For example, the skilled person may vary the proportion of lignocellulose biomass provided in the mixing and measure the resulting ethanol yield.

To determine whether the above method results in an increased ethanol yield, the parameter to be evaluated may be the amount of ethanol produced from given amounts of the lignocellulosic-derived material and the sugar product.

In the above method, an amount of lignocellulose biomass is pretreated, hydrolyzed and fermented, and an amount of the sugar product is added in any stage provided that it is present in the fermentation. The yield of such a method involving common fermentation may be compared to a reference method involving separate fermentation. Such a reference method, the same type of lignocellulose biomass is pretreated, hydrolyzed and fermented in the same way, but no sugar product is added. Instead, the same amount of the same type of sugar product is fermented separately in the reference method. The amounts provided in the reference method are the same as in the method which ethanol yield is to be compared. Further, in the comparison, the same type of fermenting agent is used in each case. Thus, as far as possible, the conditions of the method which yield is to be compared and the reference method are the same. If the amounts are successfully controlled, the ethanol yield of the method involving common fermentation will be higher than that of the reference method involving two separate fermentations.

Thus, in examples of the method of the first, non-claimed, configuration of the first, non-claimed, aspect, the amounts may be controlled such that the ethanol yield is higher than what would be the case if the same amounts of the lignocellulose-derived treatment products and the sugar product are fermented separately using the fermenting agent and said lignocellulose-derived treatment products are obtained by the same treatment of the same type of lignocellulosic biomass. In such examples, the ethanol yield may be at least 1 % higher, such as at least 2 % higher, such as at least 5 % higher, such as at least 8 % higher (see for example Table 2).

The lignocellulosic treatment products frequently comprise the inhibitors furfural, acetic acid and hydroxymethylfurfural (5-(Hydroxymethyl)furfural, HMF). The inventors have realized that these components of the lignocellulosic treatment products provide at least part of the fermenting agent-stressing effect. Further, the inventors have identified certain concentration intervals of the above-mentioned inhibitors which correspond to beneficial proportions of lignocellulose-derived material in the mixture subjected to fermentation.

Also, the inventors believe that the presence of HMF and furfural affect the fermentation in a positive way, but that they become inhibiting to the yeast at rather low concentrations, around 1 g/l. Further, the inventors believe that acetic acid may provide the most valuable contribution of the three, and that the ethanol production may not be decreased by the acetic acid concentrations normally arising in lignocellulose-derived hydrolysates. However, the acetic acid concentration should preferably be kept below 8 g/l, since such a concentration or higher have been shown to inhibit fermentations. As an example, it is shown in Table 2 that an acetic acid concentration of 0.72 g/l, a furfural concentration of 0.66 g/l and a HMF concentration of 0.15 g/l correspond to a particularly increased yield.

Thus, as a second, non-claimed, configuration of the first, non-claimed, aspect, there is provided a method of improving the ethanol yield in production of an ethanol-containing product from a lignocellulosic biomass and a sugar product containing fermentable sugars derived from a sugar-rich material, comprising:

> treatment, involving hydrolysis, of said lignocellulosic biomass in one or more steps to obtain lignocellulose-derived treatment products including fermentable sugars; and
> fermentation, using a fermenting agent, of a mixture comprising at least part of said lignocellulose-derived treatment products and said fermentable sugars derived from said sugar-rich material to obtain the ethanol-containing product, wherein an amount of said sugar product is mixed with an amount of at least one of the following:

>> (i) lignocellulose-derived material in the treatment;
>> (ii) lignocellulose-derived treatment products from the treatment; and
>> (iii) lignocellulose-derived treatment products in the fermentation,

such that said fermentable sugars derived from said sugar-rich material and said at least part of said lignocellulose-derived treatment products are present in the mixture, and said amounts are controlled such that the mixture comprises

> a) furfural in a concentration of 0.1 to 1.1 g/l, acetic acid in a concentration of 0.2 g/l or higher and HMF in a

EP 2 240 591 B2

concentration of 1.0 g/l or lower or
b) furfural in a concentration of 1.1 g/l or lower, acetic acid in a concentration of 2 g/l or higher and HMF in a concentration of 1.0 g/l or lower.

For example, the amounts may be controlled such that the concentrations are within the above-mentioned ranges in the initial phase of the fermentation, in particular if the mixing has taken place before the fermentation. Alternatively, if the sugar product is added during the fermentation, the amounts may be controlled such that the concentrations are within the above-mentioned ranges after the whole amount of the sugar product has been added.

In examples of the case in which a) applies, the furfural concentration is from 0.2 to 0.9 g/l, the acetic acid concentration is from 0.35 to 8.0 g/l or higher and/or the HMF concentration is from 0.015 to 0.75 g/l.

In examples of the case in which b) applies, the furfural concentration is 0.9 g/l or lower, the HMF concentration is from 0.015 to 0.75 g/l and the acetic acid concentration is not higher than 8 g/l.

Thus, the mixture is preferably in the form of a solution or suspension.

The amounts provided in the method may also be controlled such that a certain proportion of the fermentable sugars that are subjected to fermentation are derived from the lignocellulosic biomass.

Thus, as a third, non-claimed, configuration of the first, non-claimed, aspect, there is provided a method of improving the ethanol yield in production of an ethanol-containing product from a lignocellulosic biomass and a sugar product containing fermentable sugars derived from a sugar-rich material, comprising:

treatment, involving hydrolysis, of said lignocellulosic biomass in one or more steps to obtain lignocellulose-derived treatment products including fermentable sugars; and
fermentation, using a fermenting agent, of a mixture comprising at least part of said lignocellulose-derived treatment products and said fermentable sugars derived from said sugar-rich material to obtain the ethanol-containing product, wherein an amount of said sugar product is mixed with an amount of at least one of the following:

(i) lignocellulose-derived material in the treatment;
(ii) lignocellulose-derived treatment products from the treatment; and
(iii) lignocellulose-derived treatment products in the fermentation,

such that said fermentable sugars derived from said sugar-rich material and said at least part of said lignocellulose-derived treatment products are present in the mixture, and said amounts are controlled such that 20-80 % by weight of the fermentable sugars in the mixture are lignocellulose-derived.

In sugars canes, about 50 % of the fermentable sugars are available as lignocellulosic biomass and about 50 % as sugar. Further, the inventors believe that it may be beneficial if the majority of the fermentable sugars are derived from the sugar-rich material, e.g. the extract in the case of sugar canes. See also table 2.

Thus, in some examples, said amounts are controlled such that 30-70 % by weight of the fermentable sugars in the mixture are lignocellulose-derived.

In further examples, said amounts are controlled such that 30-60 % or 40-60 % by weight of the fermentable sugars in the mixture are lignocellulose-derived.

In examples of the first, non-claimed, aspect, the hydrolysis of the treatment may comprise enzymatic hydrolysis. Further, in such examples the hydrolysis and fermentation may be performed simultaneously, e.g., in a common vessel. Simultaneous hydrolysis and fermentation (or simultaneous saccharification and fermentation) is sometimes referred to as "SSF" herein. In SSF, the fermentable sugars produced by the enzymes are continuously fermented by the fermenting agent, and hence the sugar concentration is kept low. This is beneficial since many saccharification enzymes are inhibited by the produced sugar, i.e., their own product. Another benefit is that the fermenting agent may detoxify the solution/ suspension to some extent, which improves the enzymatic hydrolysis.

Also, in examples of the first, non-claimed, aspect, the lignocellulosic biomass may comprise sugarcane bagass and optionally sugarcane trash and thesugar-rich material may bean extract comprising canesugar. Consequently, different parts of one plant (e.g., the sugarcane) may be used a starting material in the method, which may result in a high ethanol yield per acre.

Accordingly, the method may further comprise extraction of sugar canes to obtain said sugarcane bagass and said extract. Provided sugar canes may be prepared before such an extraction. For example they may be chopped up to facilitate an efficient extraction.

In general, it is beneficial to add the sugar product late in the method. For example, if added before a pretreatment or the hydrolysis, the sugars of the sugar product may be degraded during such method steps.

Thus, in examples of the method of the first, non-claimed, aspect, the amount of said sugar product is mixed with an amount of:

(ii) lignocellulose-derived treatment products from the treatment; and/or
(iii) lignocellulose-derived treatment products in the fermentation.

In the case where simultaneous hydrolysis and fermentation SSF is employed, it may be beneficial to add the sugar product continuously throughout the SSF because the hydrolytic enzymes may be sensitive to high sugar concentrations. Further, such continuous addition is preferably initiated after the initial phase of the SSF.

Further, independent of whether SSF is used or not, it may be beneficial to add the sugar product continuously throughout at least part of the fermentation because pentoses, such as xylose, are fermented to a higher degree if hexoses and disaccharides are present, but at a low concentration. Most fermenting agents convert hexoses and disaccharides to ethanol at a higher rate than they convert pentoses. However, if the relative concentration of pentoses is allowed to be high during a period of the fermentation, the pentoses will have a competitive advantage during such a period and therefore be converted to ethanol at a higher degree than what otherwise would be the case, and the overall ethanol yield will be increased.

For example, the sugar product may be added such that the glucose concentration is kept between 1 and 5 g/l, such as between 2 and 3 g/l, in the fermentation or the SSF. In batch processes, the glucose concentration may be kept within such ranges during at least 50 % of the retention time, such as during at least 75 % of the retention time.

Thus, in examples of method of the first, non-claimed, aspect, the fermentation takes place in a vessel for fermentation and optionally hydrolysis, the amount of said sugar product is mixed with the amount of said cellulose-derived treatment products in said vessel and at least 75 % by weight of the amount of the lignocellulose-derived treatment products is added to the vessel before any addition of the sugar product. This may be done such that the glucose concentration is within the above-mentioned ranges.

In an example, at least 90 % by weight of the amount of the lignocellulose-derived treatment products is added to the vessel before any addition of the sugar product.

The same concept as described above may also be applied on the case wherein the first starting material is a lignocellulosic biomass and the second starting material is a starch-rich biomass.

Thus, as a first configuration of a second aspect, there is provided a method according to claim 1.

Consequently, one or more of the steps of the first and the second treatment may be common. That is, all the steps of the first and the second treatment up to the mixing are separate, and the steps following the mixing are common. Consequently, material derived from the starch-rich biomass and lignocellulose-derived material may be hydrolyzed separately or in common. Thus, in some embodiments of the first configuration of the second aspect, the first and the second treatment are performed separately, and the stream containing material derived from the starch-rich biomass and the stream containing lignocellulose-derived material are merged in, or just before, the fermentation.

The reasoning regarding the first, non-claimed, aspect and the various examples of the first, non-claimed, aspect apply *mutatis mutandis* to the second aspect.

"Starch-rich biomass" refers to biomass in which the majority of the saccharides are in the form of starch. Examples of starch-rich biomass are corn cobs and grains. As with the above-mentioned sugar product, a hydrolysate of a starch-rich material does normally not comprise the stress-inducing products to such an extent that the fermenting agent isstimulated to an increased ethanol production. Thus, if the amounts are controlled properly, an increased ethanol production is obtained when the starch-derived material are fermented together with the lignocellulose-derived material.

To determine whether the method results in an increased ethanol yield, the parameter to be evaluated may be the amount of ethanol produced from given amounts of the lignocellulosic-derived material and the sugar product. This is in accordance with what is described in connection with the first aspect.

Thus, the amounts of the first configuration of the second aspect may be controlled such that the ethanol yield is higher than what would be the case if the same amounts of said lignocellulose-derived treatment products, obtained by the same first treatment of the same lignocellulosic biomass, and said starch-derived fermentable sugars, obtained by the same second treatment of the same type of starch-rich material, are fermented separately using the fermenting agent. In such embodiments, the ethanol yield may be at least 1 % higher, such as at least 2 % higher, such as at least 5 % or higher, such as at least 8 % higher (see for example Table 2).

In line with what is described in connection with the first aspect, there is provided, as a second, non-claimed, configuration of the second aspect, a method of improving the ethanol yield in production of an ethanol-containing product from a lignocellulosic biomass and a starch-rich biomass, comprising:

a first treatment, involving hydrolysis, of the lignocellulosic biomass in one or more steps to obtain lignocellulose-derived treatment products including fermentable sugars;

a second treatment, involving hydrolysis, of the starch-rich biomass in one or more steps to obtain starch-derived fermentable sugars;

fermentation, using a fermenting agent, of a mixture comprising at least part of said lignocellulose-derived treatment products and at least part of said starch-derived fermentable sugars to obtain the ethanol-containing product,

wherein an amount of lignocellulose-derived material and an amount of material derived from the starch-rich biomass are mixed in the fermentation or earlier such that the at least part of said lignocellulose-derived treatment products and the at least part of said starch-derived fermentable sugars are present in the mixture, and said amounts are controlled such that the mixture comprises

a) furfural in a concentration of 0.1 to 1.1 g/l, acetic acid in a concentration of 0.2 g/l or higher and HMF in a concentration of 1.0 g/l or lower or
b) furfural in a concentration of 1.1 g/l or lower, acetic acid in a concentration of 2 g/l or higher and HMF in a concentration of 1.0 g/l or lower.

For example, the amounts may be controlled such that the concentrations are within the above-mentioned ranges in the initial phase of the fermentation, in particular if the mixing has taken place before the fermentation. Alternatively, if the starch-derived product is added during the fermentation, the amounts may be controlled such that the concentrations are within the above-mentioned ranges after the whole amount of the starch-derived product has been added.

In examples of the case in which a) applies, the furfural concentration is from 0.2 to 0.9 g/l, the acetic acid concentration is from 0.35 to 8 g/l or higher and/or the HMF concentration is from 0.015 to 0.75 g/l.

In examples of the case in which b) applies, the furfural concentration is 0.9 g/l or lower, the HMF concentration is from 0.015 to 0.75 g/l and/or the acetic acid concentration is not higher than 8 g/l.

Thus, the mixture is preferably in the form of a solution or suspension.

And further, as a third, non-claimed, configuration of the second aspect, there is provided a method of improving the ethanol yield in production of an ethanol-containing product from a lignocellulosic biomass and a starch-rich biomass, comprising:

a first treatment, involving hydrolysis, of the lignocellulosic biomass in one or more steps to obtain lignocellulose-derived treatment products including fermentable sugars;
a second treatment, involving hydrolysis, of the starch-rich biomass in one or more steps to obtain starch-derived fermentable sugars;
fermentation, using a fermenting agent, of a mixture comprising at least part of said lignocellulose-derived treatment products and at least part of said starch-derived fermentable sugars to obtain the ethanol-containing product, wherein an amount of lignocellulose-derived material and an amount of material derived from the starch-rich biomass are mixed in the fermentation or earlier such that the at least part of said lignocellulose-derived treatment products and the at least part of said starch-derived fermentable sugars are present in the mixture, and said amounts are controlled such that 20-80 % by weight of the fermentable sugars in the mixture are lignocellulose-derived.

Various proportions in a mixtures of a starch-rich and a cellulosic material are shown in Table 2.

Thus, in some examples, said amounts are controlled such that 30-70 % by weight of the fermentable sugars in the mixture are lignocellulose-derived.

In further examples, said amounts are controlled such that 30-60 % or 40-60 % by weight of the fermentable sugars in the mixture are lignocellulose-derived.

The method of the second aspect may comprise the features of the first and the third configuration.

In the first configuration of the second aspect, the hydrolysis of the first treatment and optionally the second treatment comprises enzymatic hydrolysis.

Further, in embodiments of the first configuration of the second aspect, the hydrolysis and the fermentation of the first and the second treatment are performed simultaneously (i.e. SSF), e.g., in a common vessel. Thus, in such embodiments, the lignocellulose-derived material and the material derived from starch-rich biomass is mixed before the hydrolysis. The benefits of such embodiments are described above. The sugar concentration in such a SSF may be controlled by the provided amount of starch-hydrolyzing enzymes, e.g. amylase. Consequently, the addition of the starch-hydrolyzing enzyme(s) may be adjusted such that the glucose concentration in the SSF is within the ranges mentioned above in connection with the first, non-claimed, aspect.

Also, in embodiments of the first configuration of the second aspect, the lignocellulosic biomass may be straw and the starch-rich material may be grain. Consequently, the two starting materials of the method may be provided by the same plant, which results in a high ethanol yield per acre.

The first and/or the second treatment may comprise pretreatment before the hydrolysis. Such pretreatment may be particularly beneficial in the first treatment, since the constitution of the lignocellulosic biomass makes it harder to hydrolyze. Thus, in embodiments of the first configuration of the second aspect, the first treatment may comprise pretreatment before the hydrolysis and the mixing may take place after the pretreatment.

In embodiments of the first configuration of the second aspect, the method may comprise an on-line measurement of fermentation-related parameter to obtain a value, which value is used when controlling the amounts. The concentration of one or more of the cellulose-derived treatment products is an example of such a parameter. The measurement may for example take place during the fermentation. As an example, the concentration of acetic acid may be measured in the fermentation to obtain an acetic acid value. If such an acetic acid value is lower than a predetermined reference value, the proportion of lignocellulose-derived material may be increased in the mixing and/ or if the acetic acid value is higher than another predetermined reference value, the proportion of lignocellulose-derived material in the mixing may be decreased. In a similar manner, a more complex value may be measured and correlated to the amounts provided in the mixing. For example, such complex values may be measured by NIR spectroscopy. The system of the fifth, non-claimed, aspect may be designed accordningly.

It follows from what is described above that a lignocellulose-derived material may be used for increasing the fermentability of a sugar product or a material derived from a starch-rich biomass. The addition of lignocellulose-derived treatment products to a solution or suspension containing sugar product-derived or starch-derived fermentable sugars increases the fermentability of the solution or suspension.

Thus, as a third, non-claimed, aspect of the present disclosure, there is provided a use of a lignocellulose-derived material comprising substances capable of stressing the fermenting agent for improving the fermentability of a fermentable product derived from a starch-rich biomass or a sugar product containing fermentable sugars derived from a sugar-rich material.

In the context of the third, non-claimed, aspect, the "lignocellulose-derived material" is an optionally pretreated and optionally hydrolyzed lignocellulosic biomass which comprises lignocellulose-derived treatment products or is capable of releasing lignocellulose-derived treatment products during pretreatment and/or hydrolysis.

For example, the lignocellulose-derived material of the third, non-claimed, aspect may be a lignocellulose-derived hydrolysate comprising the furfural, acetic acid and/or HMF.

Further, the fermentable product of the third, non-claimed, aspect may be a solution or a suspension. For example, the fermentable product may be the juice from a sugar cane extraction or optionally diluted molasses.

The "fermentability" refers to the extent of which something can be fermented to ethanol. The fermentability may be measured according to any one of the methods described below. Preferably, a method according to Öhgren et al. is employed. The person skilled in the art understands how to adapt the fermentability measurement method described therein to the context of the present disclosure. For example, the method may be adapted as follows:

The product (such as a solution or a slurry) which fermentability is to be measured is filtrated and the pH is adjusted to 5.5 with 20% $Ca(OH)_2$ solution. Then, the concentration of fermentable sugars is adjusted, either by addition of glucose or by dilution, to 50 g/l. The fermentation experiments are all performed in duplicate. A yeast is used in the fermentability experiments at an initial concentration of 5 g DM/L. The yeast may be *Saccharomyces Cerivisiae* (such as ordinary baking yeast from Jästbolaget, Rotebro, Sweden) or any other yeast which may be employed according to the present disclosure and which the skilled person finds suitable. Glass flasks of 25 mL with a working volume of 20 mL is used to ferment a mixture consisting of 18.5 mL filtrate and 1 mL inoculum (containing 100 g dry matter yeast/L). A volume of 0.5 mL nutrients is added to give a final concentration of 0.5 g/l $(NH_4)_2HPO_4$, 0.025 g/l $MgSO_4 \cdot 7H_2O$, 0.1 mol/L $NaH_2PO_4$, and 1 g/l yeast extract. The flasks are sealed with rubber stoppers through which hypodermic needles are inserted for the removal of the $CO_2$ produced and to take samples. The flasks are incubated at 30°C for 24 h and samples may be withdrawn after 0, 2, 4, 6, 8 and 24 h and analysed for ethanol and optionally glucose. The fermentability is determined by measuring the ethanol concentration in the sample after 24 h.

The addition of the lignocellulose-derived material, which give rise to fermenting agent-stimulating lignocellulose-derived treatment products in the product to be fermented, results in an increased fermentability of the product.

In a preferred example of the third, non-claimed, aspect, the fermentable product is a sugar cane extract or cane sugar molasses and the lignocellulose material is a sugar cane bagass hydrolysate.

An intermediate product in the production of the ethanol-containing product is the composition subjected to the fermentation. Such a composition comprises the fermenting agent-stimulating lignocellulose-derived treatment products.

Thus, as a first, non-claimed, configuration of a fourth, non-claimed, aspect of the present disclosure, there is provided a composition comprising

lignocellulose-derived hydrolysate comprising fermentable sugars and
a sugar product comprising fermentable sugars, said sugar produced being and extract from sugar cane, sugar beet or sweet surghum, or molasses made therefrom,
wherein said amount of said lignocellulose-derived is such that 20-80% by weight of fermentable sugar sof the composition are lignocellulose-derived, and wherein the lignocellulose-derived hydrolysate further comprises furfural, acetic acid and/or HMF.

As described above, the fermentability may be measured according to anyone of the methods described below.

The fermentability of the composition and the sugar product may preferably be fermented in parallel according to the adapted protocol outlined above. A relative fermentability may be determined by comparing ethanol concentration in a sample from the composition after 24 h with the ethanol concentration in a sample from the sugar product after 24 h.

In general, the fermentability of a mixed slurry (containing both lignocellulose-derived material and material derived from sugar-high material or starch-high material) may be compared to the fermentability of a pure slurry (containing material derived from sugar-high material or starch-high material) to show an increased ethanol yield.

The lignocellulose-derived treatment products may comprise the inhibitors furfural, acetic acid and/or HMF, and the proportions of the components of the composition may be defined by the inhibitors concentrations. This is further discussed above in connection with the first, non-claimed, aspect.

Thus, as a second, non-claimed, configuration of the fourth, non-claimed, aspect, there is provided a composition comprising lignocellulose-derived treatment products comprising lignocellulose-derived fermentable sugars and a sugar product comprising fermentable sugars derived from a starch-rich or sugar-rich material, wherein the amount of the lignocellulose-derived treatment products is such that the composition comprises

a) furfural in a concentration of 0.1 to 1.1 g/l, acetic acid in a concentration of 0.2 g/l or higher and HMF in a concentration of 1.0 g/l or lower or

b) furfural in a concentration of 1.1 g/l or lower, acetic acid in a concentration of 2 g/l or higher and HMF in a concentration of 1.0 g/l or lower.

In a composition according to the second, non-claimed, configuration of the fourth, non-claimed, aspect, substantially all of the furfural, acetic acid and HMF will normally be provided by the lignocellulose-derived treatment products since the concentrations of such substances are very low in the sugar product of the fourth, non-claimed, aspect.

In some examples wherein a) applies, the furfural concentration may be 0.2 to 0.9 g/l, the acetic acid concentration 0.35 g/l to 8 g/l and/or the HMF concentration 0.015 to 0.75 g/l.

In some examples wherein b) applies, the furfural concentration may be 0.9 g/l or lower, the HMF concentration 0.015 to 0.75 g/l and/or the acetic acid concentration not higher than 8 g/l.

Also, the amount of sugar provided by each component if the composition may also define the relation between them.

Thus, as a third, non-claimed, configuration of the fourth, non-claimed, aspect there is provided a composition comprising lignocellulose-derived treatment products comprising lignocellulose-derived fermentable sugars and a sugar product comprising fermentable sugars derived from a starch-rich or sugar-rich material, wherein the amount of the lignocellulose-derived treatment product is such that 20-80 % by weight of the fermentable sugars of the composition are lignocellulose-derived.

The remaining fermentable sugars may be derived from the starch-rich or sugar-rich material, but also from another component which is not lignocellulose-derived.

In examples, 30-70 % by weight, such as 30-60 %, such as 40-60 % by weight, of the fermentable sugars of the composition are lignocellulose-derived.

In examples, the lignocellulose-derived treatment products may be a lignocellulose-derived hydrolysate, such as a bagass-derived hydrolysate.

As a fifth, non-claimed aspect of the present disclosure, there is provided a system for the production of an ethanol-containing product from sugar canes. For example, the system may be used when the method of the first, non-claimed, aspect or the use of the third, non-claimed, aspect is put into practice.

The system for the production of an ethanol-containing product from sugar canes comprises

an extraction unit for the extraction of optionally disintegrated sugar canes to obtain sugarcane bagass and an extract comprising cane sugar;

a pretreatment unit for pretreating the bagass to obtain pretreated bagass;

a first transportation arrangement for transporting the bagass, which first transportation arrangement is connected to the extraction unit and the pretreatment unit;

a hydrolysis and fermentation arrangement for separate or simultaneous hydrolysis and fermentation, in which the ethanol-containing product is produced;

a second transportation arrangement for transporting the pretreated bagass, which second transportation arrangement is connected to the pretreatment unit and the hydrolysis and fermentation arrangement;

a cane sugar inlet for receiving the extractor sugar product derived there from, which inlet is arranged on said pretreatment unit, said first transportation arrangement, said hydrolysis and fermentation arrangement or said second transportation arrangement such that said extract or cane sugar product may be subjected to fermentation,

a third transportation arrangement for transporting the extract, on which one or more sugar processing units and/or a cane sugar reservoir for holding the extract or a sugar product derived therefrom are optionally arranged, which third transportation arrangement is connected to the extraction unit and the cane sugar inlet,
wherein said first transportation arrangement is different from said third transportation arrangement.

The hydrolysis and fermentation arrangement may comprise two separate vessels for hydrolysis and fermentation, respectively. A separate hydrolysis vessel may be adapted for enzymatic or acidic hydrolysis. In the latter case, the hydrolysis vessel should preferably be designed to withstand high pressures, high temperatures and low pH. Alternatively, it may comprise a single vessel for simultaneous hydrolysis and fermentation. In such case, the vessel may be adapted for enzymatic hydrolysis.

As explained above, it may be beneficial to provide the sugar product throughout the fermentation process to control the concentration of fermentable sugars in the fermentation. Thus, in examples of the fifth, non-claimed, aspect, the cane sugar inlet is arranged on said hydrolysis and fermentation arrangement or said second transportation arrangement. If the hydrolysis and fermentation are performed in separate vessels, the cane sugar inlet is preferably arranged down-stream of the hydrolysis vessel.

In examples of the fifth, non-claimed, aspect, the system may further comprise a disintegrator for disintegration of sugar canes, which disintegrator is connected to said extraction unit.

The system may be adapted for pentose fermentation and/or low sugar concentrations during enzymatic hydrolysis. Thus, in examples of the fifth aspect, said hydrolysis and fermentation arrangement may comprise a fermentation vessel for producing the ethanol-containing product and optionally a separate hydrolysis unit for hydrolysis of the pre-treated bagass,

said sugar inlet is arranged on said fermentation vessel and
a second inlet for receiving bagass-derived material is arranged on said fermentation vessel, said second inlet being connected to said second transport arrangement, optionally via the hydrolysis unit.

Also, some of the bagass produced may be used for production of steam, which in turn may be applied in a distillation of the ethanol-containing product.

Thus, in examples of the fifth, non-claimed, aspect, the system may further comprise
a boiler for steam production, which boiler is connected to said extraction unit such that it may receive bagass and a distillation unit for distillation of the ethanol-containing product, wherein the boiler is connected to the distillation unit such that the distillation unit may receive steam from the boiler.

Below, some findings of the present disclosure are described in more detail, sometimes referring to the above-mentioned items.

The above-mentioned expression "fermentability" refers to how much of the fermentable sugars in a solution which are converted to ethanol within 24 hours under standard conditions. The measurement is performed by a standard method. This standard method is described in the literature at many places, for example in App. Biochem. Biotech. 2002, 98-100, 5-21; Söderström et. al., in Biomass Bioenergy 2003, 24, 475-486; Söderström et. al., and in App. Biochem. Biotech. 2005, 121-124, 1055-1067; Ohgren et al. The person skilled in the art understands how to adapt the standard method according to any one of the literature references to the context of the above items.

Preferably, the material flows are mixed such that a stress factor in the range of 1.05 to 1.20 is obtained.

However, as the skilled person understands, an upper limit for the stress factor is not essential to the method. A stress factor exceeding 1.25 may of course be beneficial.

As indicated before, the stress factor has a direct coupling to the ethanol yield of the fermentation of described mixture.

In degradation of lignocellulosic material, a solution/suspension is obtained which comprises pentoses as well as hexoses (pentoses from the hemicellulose and hexoses from the hemicellulose and above all cellulose in the form of glucose). In addition to pentoses and hexoses, the solution comprises a large number of other chemical substances, of which some are sometimes called inhibitors. This is because these substances, when a certain concentration is exceeded, are inhibiting the yeast, first leading to reduced growth and at yet higher concentrations to decreasing ethanol yield. The ethanol yield can decrease to zero at a much too high concentration of a certain inhibitor or inhibitors in mixture. A few inhibitors are known, such as acetic acid, furfural and HMF. There are probably a number of inhibitors which are not yet discovered in the sense that their chemical formula is not known. It is shown herein, that if inhibitors emanating from lignocellulosic material in a suitable concentration, i.e. in an amount which is not too high, are allowed to be present in a saccharide-containing solution/suspension, they east will be affected such that more ethanol is produced from a certain amount of saccharides, i.e. the yeast is stressed with the described result. Due to the stress, the yeast has to produce more energy, ATP (adenosintriphosphate), which it will get by producing ethanol. The core of the above items is to mix saccharide material A, derived from lignocellulosic material, with its content of inhibitor(s), in the right proportion with the right proportion of saccharide material according to B and/or C. When saccharides are obtained from starch-rich material as well as sugar-rich material, water is used for dilution, etc. The more of this water that is replaced by saccharide-containing solution/suspension derived from lignocellulosic material, the less energy is consumed for producing a certain amount of ethanol. This is because the higher the concentration of the ethanol-containing product after fermentation, i.e. the less water present in the ethanol-containing solution, the less energy is needed to increase the temperature of the solution to the vaporization or distillation temperature. If then all water from the mash is removed to obtain a dry, solid residue further energy savings will be achieved. If a conventional one line-process for recovery of saccharides from lignocellulosic material is compared to the method according to the above items, a larger amount of saccharides in the solution or suspension which is derived from the lignocellulosic material according to the above items is obtained than if no mixing of the material flows took place. Altogether, this leads to energy savings.

The starting material for the saccharide recovery has been exemplified above. Further, it is described above that the saccharide recovery, at least to a beginning, takes place within at least two different production lines.

The at least two material flows are merged and are forming a mixture, just before or in the fermentation step at the latest. Referring to the items, the at least two material flows preferably are merged and forming a mixture in a treatment step before the fermentation step.

The ethanol production from sugar-rich material, such as sugar canes, may take place in the following way. In the first step in the process, the sugar canes are chopped. Thereafter, the sugar cells in the chopped material are demolished by means of, for example, roller mills with the addition of water to extract as much of the sugar as possible without diluting the sugar juice too much. The solids, i.e. the bagass, are separated from the sugar juice, which is thereafter sterilized by heating to the boiling point, whereupon lime diluted in water is added. The sugar juice can rest for 30 minutes so possible particles in the juice are allowed to sediment. Consequently, the lime is added to facilitate the sedimentation. Thereafter, the juice is concentrated from approximately 13% sugar by weight to approximately 18% sugar by weight, which it should have during a certain storage time before the fermentation. The concentration is achieved by means of vaporization. It happens that the juice is slightly diluted with water before the fermentation step.

In ethanol production from another sugar-rich materials, such as sugar beets, the molasses originating from conventional saccharide recovery is used as raw material. Such molasses has a sugar concentration of above 50 % by weight and stands long time storage without risk of infection.

The saccharide recovery in one of the lines can take place according to the above, also in the method according to the above items. However, in a preferred example of the above items, at least one of the water additions described above is replaced by an addition of saccharide-containing solution/suspension from the other line of saccharide recovery, i.e. the material flow which emanates from the lignocellulosic material.

Ethanol production from starch-rich material, such as corn cobs, can occur in the following way. The first step is that the corn cobs are milled to a fine powder in a mill. Then the starch powder is mixed with preheated water to a dry content of approximately 35 percentage of weight. The starch suspension is heated to a temperature of about 90 to 95°C, and enzymes dissolved in water are then added. Said suspension is introduced in something called liquefaction tanks where enzymatic degradation leads to a reduction in the amount of water insoluble material in the suspension. The retention time in the liquefaction is usually between two and four hours. After the liquefaction, the temperature is reduced to approximately 60°C and additional enzyme in the form of a water solution is added. Here, the soluble starch is degraded to fermentable saccharides. A minor amount of material foreign to the species, such as lignocellulosic material, can accompany the starch powder. In those cases it might be preferable to also add an enzyme (or many enzymes, enzyme mixture) which degrades the cellulose in the lignocellulosic material to saccharides. In the first place, this is in order to decrease the viscosity of the hydrolyzed fluid. This fluid is later fermented by added yeast at a temperature of about 35°C. Since the enzymes are still present in the fluid, the enzymatic hydrolysis continues during the fermentation step.

The above-mentioned line for saccharide recovery, i.e. up to the fermentation step, can be useful in the method according to the above items. Though, it is preferable according to the above items, that at least one of the water additions, as described above, is replaced by an addition of saccharide-containing solution/suspension from another line of saccharide extraction, in the form of a material flow which emanates from lignocellulosic material.

Regarding the recovery of saccharides from lignocellulosic material, for example in the form of stover, straw or bagasse, it may be performed in many different manners. Here, only a few ways are described.

One in the literature often described method is acid hydrolysis. It means that to the lignocellulosic material, after it has been pulverized, an acidic water solution, i.e. a water solution with a relatively low pH-value, is added. Hydrogen ions can be added in the form of an organic or inorganic acid. Mineral acids are of frequent occurrence and the most common acid among them is sulphuric acid and sulphurous acid (which for example originates by dissolving the gaseous sulphur dioxide in water). The treatment usually takes place at increased temperature and increased pressure and may comprise one or two steps. In recovery of saccharides from the lignocellulosic material wood, two steps are recommended: one initial relatively mild step, in which primarily hemicellulose is converted to fermentable saccharides and a following step, which is more drastic in view of temperature and pressure, in which the cellulose is converted to glucose. In recovery of saccharides from the lignocellulosic materials which are exemplified above, one step is usually enough. However, regarding bagass, two steps may be preferred. If the hydrolysis conditions in the step are powerful, the hemicellulose as well as the cellulose is converted to monosaccharides. In the light of that the degraded saccharides are dissolved and the lignin is primarily in a solid form, the material flow can be designated "suspension" with high water content. The material flow can be designated "solution" if the part of the solids is removed in any position, for example by filtration. A gentle hydrolysis procedure can be combined with a second step, where a water solution comprising enzymes is added to the partly degraded lignocellulosic material. A special case of such a saccharide recovery procedure consists of that the enzymatic hydrolysis and the fermentation are performed simultaneously in the same reaction vessel, i.e. SSF (abbreviation described earlier). There are several advantages with such a procedure. The primary advantage is that the yeast ferments the sugar to ethanol as soon as the sugar is set free, and since monomer sugar has a negative effect on the enzymes activity if the sugar concentration is too high, the enzymes will in this way work considerably much more effective.

For example, this is applicable to the method according to the above items after the above-mentioned mixing of material has taken place.

A number of fermenting agents can be used in the fermentation step, for example different types of yeast. A preferred yeast type is the naturally occurring Saccharomyces cerevisiae or a modified variant thereof. Though, the methods of the present disclosure are working for all micro organisms which give an increased ethanol yield when stressed by the substances that are produced by the pretreatment/ hydrolysis of lignocellulosic biomass.

Advantage

In all processes where the whole crop is used as raw material for ethanol production, for example both the corn cob and the corn stover, the cultivated earth is utilized to a high degree.

When sugar is fermented to ethanol, the theoretical stoichiometric yield is 0.51 gram of ethanol per gram of sugar. In reality, this is never achieved when using for example yeast, since the yeast needs part of the energy for growth, etc. Further, a few by-products are formed by the fermentation to maintain the variety of balances which occur in the yeast cells. In industrial production of ethanol from sugar emanating from sugar canes and from sugar from starch derived from corn cobs, respectively, the ethanol yield is considered optimized already today due to the maturity of the technology. When practicing the methods and uses of the present disclosure, it is possible to further increase the ethanol yield compared to the ethanol yield which is today considered to be optimal.

Further, the present disclosure may provide for decreased energy needs (more concentrated ethanol solution in the distillation step) in the last step of the ethanol production, which decreases the production costs for ethanol. Furthermore, the risk of infection in the fermentation step is reduced if, according to the present disclosure, in one or more positions the water addition is replaced with an addition of saccharide-containing solution/suspension emanating from the lignocellulosic material. The risk of the water being contaminated is higher than in the case with said solution/suspension. Further, said solution/suspension is toxic for many infection organisms. Additionally, an addition of nutrients to the lignocellulose-derived solution/suspension will be obtained from the solution/suspension emanating from the sugar-rich material or the starch-rich material.

Description of drawings

In Figure 1, an embodiment of the method according to the above items is shown schematically.

Figure 2 illustrates a flow chart of the ethanol production when using canes as raw material and using enzymatic hydrolysis in the production. The canes are divided into two flows; bagass and juice. The two material flows with juice and bagass can be mixed at alternative positions at the latest before or during fermentation. The dotted lines show alternative routes for the juice in the ethanol production and the dashed lines show alternative routes for the bagass in the ethanol production.

Figure 3 illustrates a flow chart of the ethanol production when using canes as raw material and using acidic hydrolysis in the production. The canes are divided into two flows; bagass and juice. The two material flows with juice and bagass can be mixed at the latest before or during fermentation.

Detailed description

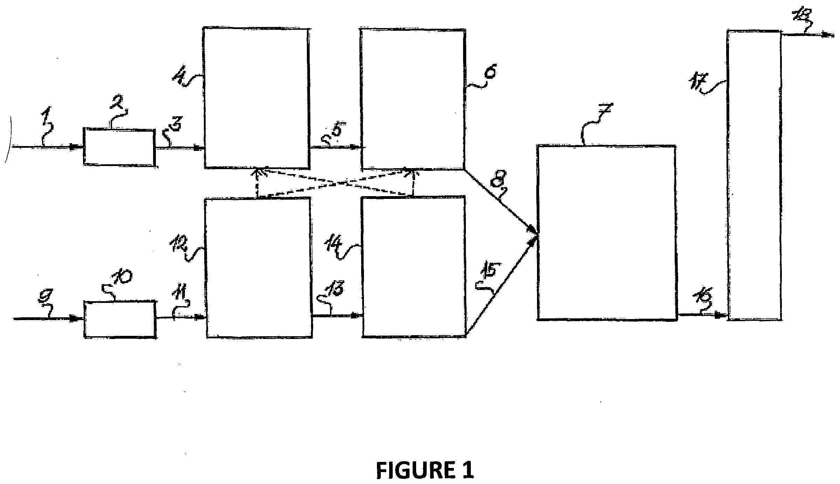

Below, an embodiment of the method according to the above items is described with reference to the flow chart according to Figure 1. In connection thereto, certain steps in the procedure are described in more detail. Finally, an embodiment example where the method of the present disclosure is simulated in a laboratory is showed.

With reference to the flow chart which is shown in Figure 1, an embodiment of the process according to the above items is described, wherein the starting material for the saccharide recovery and later the ethanol production is composed of crops, i.e. wheat, which is divided into grains and wheat straw.

The wheat is transported by a transport device 1 to a mill 2 where the wheat flour is produced. The material, which is obtained from the mill, comprises wheat flour and finely divided shell parts. The latter can be separated from the flour by bolting. Non bolted material can be used as starting material, but in the following it is described how to deal with bolted material, i.e. flour. This flour is transported in the pipe 3 to the liquefaction vessel 4. To the substantially dry wheat flour, a water solution comprising one or more enzymes is added. The function of the enzymes is to convert starch in the flour to the monosaccharide glucose. The water solution is added in an amount such that a slurry having a dry content in the range of 30-40% is formed. The temperature in the slurry is relatively high, about 80-90°C. The retention time is in the order of hours, for example 3. The pH-value in the slurry is of importance and is set to be in the range of 5-6 by means of an acid addition.

After the liquefaction, the slurry is transported by the pipe 5 to the saccharification vessel 6. The temperature of the slurry is allowed to decrease, for example to 60-70°C, and the pH-value is lowered by means of an additional acid addition to a value in the range of 4-4.5. Additional enzyme, for converting starch to glucose, is added in the form of a water solution. By doing so, the dry content of the slurry is reduced. The retention time for the slurry in the vessel 6 is determined by whether it is desired that substantially all starch is converted to glucose before the slurry/suspension is leaving the vessel or part of the starch is intact when the slurry/suspension is transported to the fermentation vessel 7. The retention time in the first case is usually in the range of 15 to 30 hours and in the second case in the range of 4 to 6 hours. The slurry is transported in the pipe 8 to the fermentation vessel 7.

The lignocellulosic part of the material, i.e. the straw, is transported by a transporting device 9 to a disintegration device 10, for example a chopping device. The straw pieces are then transported via the pipe 11 to the treatment station 12. The straw is then impregnated with an acidic solution. The impregnation can occur in many ways, for example by spraying or immerging, where upon excessive fluid is pressed out and the straw is introduced in a steam treatment vessel. The excessive fluid may be reused orcollected and purified. Different treatment temperatures and times can be chosen. A suitable temperature interval is 180-200°C. Then, the retention time can be kept short, for example 15 minutes or a few minutes less. By described treatment, it is primarily the hemicellulose in the straw which is degraded into its constituent parts, i.e. different saccharides in the form of pentoses as well as hexoses. The material, i.e. the partly degraded straw with surrounding fluid in the form of a water solution comprising a large number of released chemical substances, is transported via the pipe 13 to the hydrolysis vessel 14. The heat treatment can also be performed without acid addition and then the acetic acid which is set free from the acetyl groups of the hemicellulose is utilized. This is called auto-hydrolysis. A water solution of enzymes is added to the slurry/suspension, which enzyme shave the capability of converting the cellulose content of the straw to the monosaccharide glucose. The temperature of the hydrolysis vessel is preferably kept within the range of 40 to 55°C, but is of course dependent on the temperature at which the chosen enzymes work optimally. Regarding the retention time, it is, like in the earlier described line of saccharide recovery (using wheat as starting material), dependent on whether it is desired that substantially all cellulose is converted to glucose before the slurry/suspension is leaving the vessel or part of the cellulose is intact when the slurry/suspension is transported to the fermentation vessel 7, which takes place via the pipe 15. The retention time in the first case is usually in the range of 30 to 72 hours and in the second case in the range of 20 to 40 hours. Also these retention times are strongly dependent on which enzymes that are chosen and in which concentrations they are added. A higher quantity of and more effective enzymes result in shorter retention times. The pH during the enzymatic hydrolysis may be about 5, such as 4-6.

For said saccharides to be converted to ethanol in the fermentation vessel 7, a fermenting agent has to be added. The most common among those agents are different yeasts, and an often used yeast is of the strain Saccharomyces cerevisiae. Suitably, the yeast is dissolved in sterilized water before the addition and the fermentation takes place at a certain temperature and a certain pH. An example of temperature is 36-37°C and of pH is about 5.5. The retention time for the saccharide-containing solution/suspension in the fermentation vessel 7 is many hours. Regarding the addition of yeast, it is mainly considered in the beginning of the process, since it is long living and also reproducing. If the yeast by any reason performs poorly and/or dies, new yeast has to be added.

As is evident from the above, it is possible to exclusively convert the saccharides to ethanol in the fermentation vessel 7, i.e. only a fermentation takes place, or it is possible to perform the final conversion of described sugar polymers to monosaccharides by means of enzymes at the same time as fermentation takes place (SSF). Which method to be chosen depends on many circumstances and is determined from case to case, i.e. for each ethanol production. The following occurs chemically during the fermentation:

$$C_6H_{12}O_6 \rightarrow 2C_2H_5OH + 2CO_2 \qquad (g)$$

The fermented material in the form of mash is transported via the pipe 16 to a distillation apparatus 17. In this apparatus, the mash is heated to the boiling point of ethanol, 78.3°C, which results in that the ethanol is leaving the apparatus in the form of gas via the pipe 18. The ethanol gas is cooled down and the ethanol is obtained in the liquid state. At the same time, the remaining fluid and the solid residues (mainly lignin and/or degraded products of lignin) are drained off from the lower part of the plant (not shown in the figure). To prevent the solids from accompanying into the distillation unit 17, the mash can pass a separation unit of any kind, for example a laminated separator.

The core of the above items is that the material flows are mixed in a certain proportion of amounts at the latest in the fermentation step, which is the case in the flow chart in Figure 1. The optimal amount of each respective material flow is primarily dependent on the pretreatment/hydrolysis of the material flows. One way of determining the relation of amounts is to do preliminary laboratory studies with a certain starting material. By preparing different mixtures of the two material flows at the laboratory and then perform measurements of the ethanol yield according to the above-mentioned standard method, by the different mixtures as well as the saccharide-containing solution emanating from for example the wheat or the corn cobs, the stress factor in each case can be calculated, and if the stress factor is plotted versus the mixture a maximum regarding the stress factor will be obtained and thereby also the ethanol yield, and then the right mixture may be found. It is not compulsory to choose exactly that mixture in reality, there can be many parameters or circumstances which affect which mixture that is actually used. A circumstance of importance is the access of the respective raw material. Dependent on the crop used as starting material, a certain distribution of the lignocellulosic material (for example the straw, the stover, the bagass) and the starch-rich material or the final sugar will be at hand. Sometimes, part of the lignocellulosic material is needed for other useful purposes. For example, there are soils (growth substrate) which require that the corn stover is plowed down in the soil. If the mass balance is incorrect, for example in reaching a maximal stress factor and thereby also an optimal ethanol yield, and an excess of saccharide material from a processing line is obtained, it is easy to expel the excesses and sell it on the market. Said problem can also be solved by the addition of one or more external inhibitors, i.e. inhibitors known to stress the fermenting agent. Another way of solving said problem is, for supplementary purposes, to add a material flow of partly degraded lignocellulosic material of other origin than the exemplified above.

According to a preferred embodiment of the method according to the above items, the addition of external water is reduced or eliminated in a recovery line with saccharide-containing solution/suspension from the other recovery line. This is shown in the flow chart with dashed lines. If the material flow is withdrawn in the upper part of the treatment vessel, then it will be a water solution lacking solids, i.e. not a slurry/suspension. If looking at the flow chart which is shown in Figure 1, an alternative embodiment of the method according to the above items is found, wherein the vessels 6 and 14 are replaced by a common vessel, which is larger by volume. It means that both pipes 5 and 13 lead to one and the same vessel.

These embodiments of the method according to the above items result in that the amount of water in the mash, which is transported to the distillation column 17, is low. This, in turn result in low energy consumption in the distillation step and, in the end, contributes to a low production cost for ethanol.

*Ethanol production from sugar canes*

Sugar canes from cane fields can be used in ethanol production.

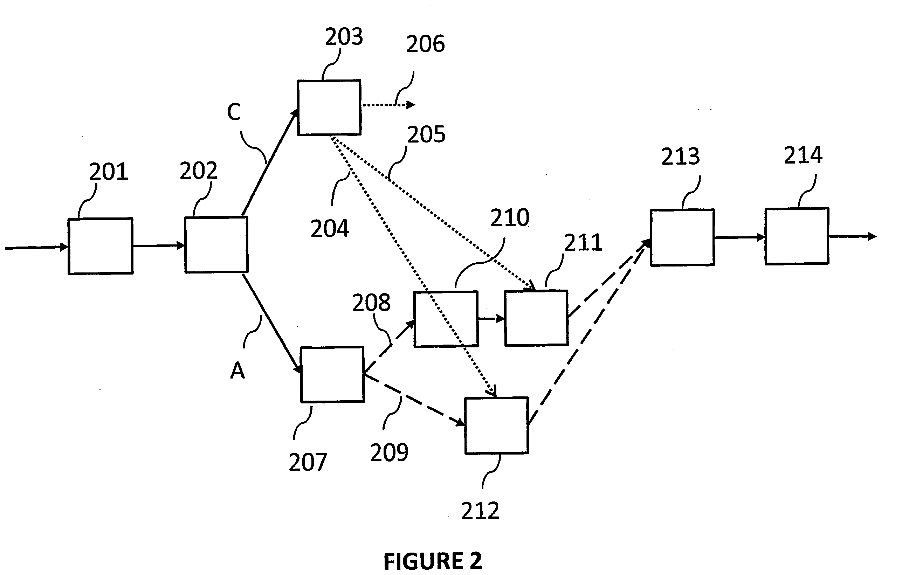

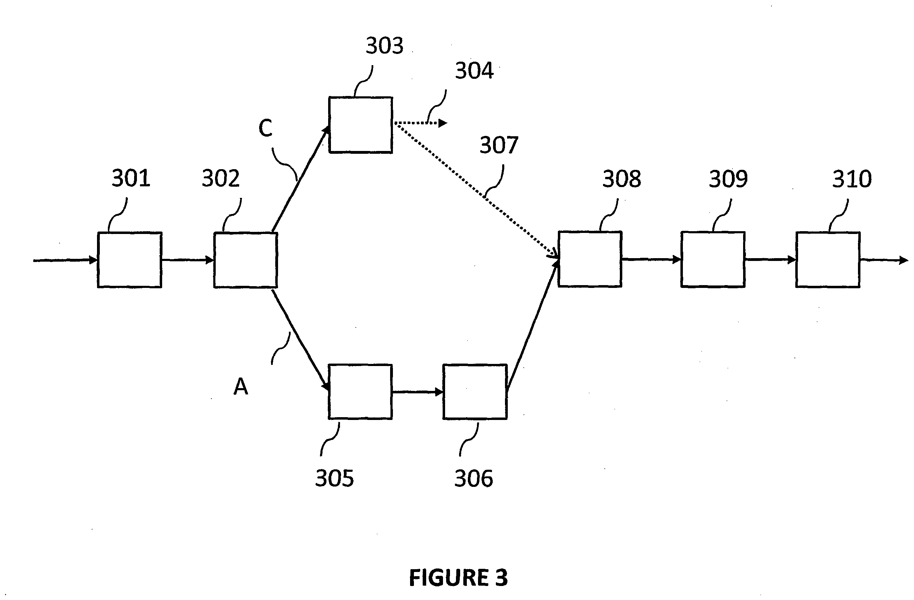

With reference to the flow charts which are shown in Figure 2 and 3, two examples of non-claimed methods according to the present disclosure are described, wherein the starting material for the saccharide recovery and later the ethanol production is sugar canes. The sugar canes are prepared and separated into bagass and juice during the process. The two flows comprising bagass-derived material and juice, respectively, are subsequently merged. The merge may take place at alternative positions along the ethanol production process.

An ethanol production line using enzymatic hydrolysis is described with reference to the flow chart shown in Figure 2, whereas an ethanol production line using an acidic hydrolysis is described with reference to the flow chart shown in Figure 3. Several process steps take place during the ethanol production and some of these steps can be performed in different order and a few of them can be excluded. Different alternatives and alternative pathways are shown in Figure 2 and 3.

*Preparation*

The sugar canes are first prepared in a preparation device 201, 301. The preparation involves disintegration of the canes. The preparation device can for example be a chopping device.

*Juice extraction*

After the preparation in the preparation device 201, 301, the prepared canes are transported to a juice extraction unit 202, 302, which may comprise rolling mills that facilitate the extraction. During the juice extraction 202, 302, the canes are separated into two parts; juice and bagass. From the juice extraction unit two flows are thus formed; one containing juice C and another containing bagass A. The juice contains sugar. The juice can optionally be dehydrated (evaporated). The dehydration forms molasses, which is a thick product of high sugar concentration. The bagass is the fibrous residue remaining after the extraction of the juice from the sugarcane stalks.

The two flows from the extraction unit can be merged via alternative routes 204, 205, 307 in the ethanol production line. Below, the processes in the ethanol production line are described in detail.

*Juice processing*

Before the juice C is mixed with the bagass-derived flow A, the juice can be subjected to further processing which is illustrated by a device 203, 303. The device 203, 303 may comprise one or more units and processes the juice (these units are not explicitly shown in Figure 2 or Figure 3). Such a process step may for example be juice clarification. The juice clarification takes place in a clarification unit, and after the juice clarification, the juice can be added to the bagass-derived flow A via various routes 204, 205, 307. An alternative of complementing step is to further process the juice in a juice evaporation step. Such juice evaporation takes place in an evaporation unit (not explicitly shown in Figure 2 and Figure 3). During the evaporation the concentration of sugar increases whereas the water content decreases. After the juice evaporation the juice can be added to the bagass-derived flow A via alternative routes 204, 205, 307. Antother alternative or complementing step is sterilization of the juice, e.g., by heating it to the boiling point. Further, a part of the juice for may be used for sugar production. In such case, the part of the juice is diverted 206, 304 from the ethanol production line and is not mixed with the bagass-derived flow A.

External sources of molasses may be used in the fermentation (not shown in Figure 2 and Figure 3). The molasses may be added to the bagass-derived flow A at alternative routes 211, 212, 308.

If the juice or molasses contains high concentrations of sugar, it may be diluted, with for example water, before being added to the bagass-derived flow A.

In some cases, the bagass-derived flow A is too concentrated after the hydrolysis and then the mixing with flow C results in a beneficial dilution of the flow A. In such cases the flow C should not be too concentrated.

Below the treatment of bagass, the mixing of flows A and C and further processing are described. First an example utilizing enzymatic hydrolysis is described and a description of an example utilizing acidic hydrolysis follows.

*Enzymatic hydrolysis*

The enzymatic hydrolysis is described with reference to Figure 2. When choosing enzymatic hydrolysis, two routes are possible. Both are described below.

*Pretreatment* of bagass

After the juice extraction, the bagass is further processed via route A. Optionally, part of the bagass may be used in steam or power production. Alternatively, or as a complement, the lignin-containing co-products of the ethanol production process may be used for the steam or power production. The power or steam may be used in the ethanol production and may hence be brought back to the ethanol production line in the form of energy (not shown in Figure 2). For example, part of the bagass may be used in production of steam, which is subsequently used in the pretreatment or distillation.

When the bagass is further processed it is transported via route A to a pretreatment unit 207. The pretreatment unit may be a vessel or a container. An effective pretreatment is needed to render the cellulose of the bagass accessible to the enzymes in the subsequent hydrolysis step. Further, the majority of the hemicellulose is normally hydrolysed to monomeric sugars during the pretreatment.

In one example, the first step in the pretreatment is impregnation. Impregnation refers to impregnating the cellulose biomass with an impregnation fluid. The impregnation fluid may be an acid solution, such as a mineral acid solution. The impregnation may be performed with acid solutions having different pH, such as a pH of 0.5-5.5, or such as a pH of 0.5-2. The impregnation may also be performed with a gas, such as a $SO_2$-gas, or with the combination of a gas and a liquid.

Alternatively, the first step of the pretreatment is steaming optionally followed by impregnation. Steaming refers to a process used to drive air out from the cellulosic biomass to facilitate further hydrolysis of the cellulose.

Further, the pretreatment may involve steam explosion. Steam explosion refers to a process that combines steam, shearing forces and hydrolysis for rupturing cellulosic fibers.

The pre-treated bagass is transported via route 208 to the hydrolysis unit 210 or via route 209 to the hydrolysis unit 212. The pretreated bagass material is neutralized before the enzymatic hydrolysis. For example, the pretreated bagass may be neutralized by means of an addition of NaOH or ammonia. Also, lime stone (CaOH) may be used, which is a cheap alternative.

*SSF Simultaneous Saccharification and Fermentation*

If selecting enzymatic hydrolysis of the pretreated bagass, one route 209 involves Simultaneous Saccharification and Fermentation, SSF. That is, the hydrolysis of the cellulose and the fermentation take place in the same unit 212.

During SSF, 212, the pre-treated bagass is, together with enzymes, feeded into the fermentation. The enzymes have the capability of converting the cellulose content of the bagass to monosaccharides. The pretreated bagass is thus hydrolysed to a bagass hydrolysate, which comprises fermentable sugars and inhibitory substances.

During the fermentation in SSF at least one fermenting agent is utilized and that agent can be yeast. The yeast may be wild type, mutant or recombinant *Saccaromyces cerevisae.* Suitably, the yeast is dissolved in sterilized water before its addition. Regarding the addition of yeast, it is mainly considered in the beginning of the process, since it is long living and also reproducing. If the yeast by any reason performs poorly and/or dies, new yeast has to be added.

The temperature of the hydrolysis and fermentation unit 212 is preferably kept within the range of 30 to 37 °C, but is of course dependent on the temperature at which the chosen enzymes and the yeast work optimally. The fermentation is an exothermic reaction, and therefore cooling is normally needed to keep the temperature within the described range.

The pH during the SSF should preferably be in the range of 4.8 to 6, more preferably 5.3 to 5.7. The pH is normally adjusted during the above-mentioned neutralization. It may be necessary to measure the pH throughout the SSF and adjust the pH if needed, partly because acetic acid is formed/released during the process.

The juice C is after the juice extraction and optionally the juice clarification and evaporation mixed with the bagass-derived flow A before or during the SSF 212 via route 204.

In the merge of the flows A and C, the proportions of the respective amounts are controlled such that the fermenting agent is stressed to an extent where an improved ethanol yield is obtained. Different proportions of the flows may be tested and the resulting ethanol yield measured. The proportion of flows resulting in the best ethanol yield is selected, and subsequently, the flows are controlled to be merged in such a proportion.

A low sugar concentration in the SSF promotes the xylose fermentation. Consequently, it may be beneficial to keep the sugar concentration low. The sugar concentration may be kept low by adding the bagass-derived material first and allowing the SSF to proceed for a while without any sugar addition. Then, the sugar is added continuously during a period such that the glucose concentration in the SSF is 2-3 g/l. Finally, the SSF is allowed to proceed for a while after the sugar addition is completed. In this configuration, the fermenting agent(s) employed is/are capable of fermenting pentoses.

*SHF- separate hydrolysis and fermentation*

The other route 208 of enzymatic hydrolysis involves two separate steps; a first hydrolysis step 210 and a second fermentation step 211.

The pre-treated bagass is transported to a hydrolysis unit 210. The pre-treated bagass is subjected to at least one aqueous hydrolysing agent, which comprises saccharification enzymes, and is hydrolysed to a bagasshydrolysate, which comprises fermentable sugars and inhibitory substances.

The temperature of the hydrolysis unit is preferably kept within the range of 40 to 50 °C, but is of course dependent on the temperature at which the chosen enzymes work optimally. The pH is preferably within the range of 4.0 to 5.6. The pH is normally adjusted during the above-mentioned neutralization.

The retention time is strongly dependent on which enzymes that are chosen and in which concentrations they are added. A higher quantity of and more effective enzymes may result in shorter retention times.

After the hydrolysis 210, the bagass hydrolysate is transported to a fermentation unit 211 and is subjected to fermentation in an aqueous liquid fermentation utilizing at least one fermenting agent. The fermenting agent can be yeast such as wild type, mutant or recombinant *Saccaromyces cerevisae.* Suitably, the yeast is dissolved in sterilized water before its addition. If the yeast by any reason performs poorly and/or dies, new yeast has to be added.

The fermentation takes place at a temperature of 30 to 37°C. and a certain pH. The fermentation is an exothermic reaction, and therefore cooling is normally needed to keep the temperature within the described range.

The pH during the fermentation should preferably be in the range of 4.8 to 6, more preferably 5.3 to 5.7. It may be necessary to measure the pH throughout the fermentation and adjust the pH if needed, partly because acetic acid is formed/released during the process.

The juice C is after the juice extraction and optionally the juice clarification and evaporation mixed with the bagass-derived flow A before or during the fermentation 211 via route 205. Thus, the merging of the flows A and C occurs after the hydrolysis 290 but at the latest during the fermentation 211.

In the merge of flows A and C, the proportions of the respective amounts are controlled such that the fermenting agent is stressed to an extent where an improved ethanol yield is obtained. Different proportions of the flows may be tested and the resulting ethanol yield measured. The proportion of flows resulting in the best ethanol yield is selected, and subsequently, the flows are controlled to be merged in such a proportion.

A low sugar concentration in the fermentor promotes the xylose fermentation. Consequently, it may be beneficial to keep the sugar concentration low. The sugar concentration may be kept low by adding the bagass-derived material first and allowing the fermentation to proceed for a while without any sugar addition. Then, the sugar is added continuously during a period such that the glucose concentration in the SSF is 2-3 g/l. Finally, the fermenting is allowed to proceed for a while after the sugar addition is completed. In this configuration, the fermenting agent(s) employed is/are capable of fermenting pentoses.

*Acidic hydrolysis*

As mentioned before, an ethanol production line may utilize acidic hydrolysis instead of an enzymatic hydrolysis. Below, the acidic hydrolysis and corresponding process steps in the ethanol production are described with reference to Figure 3.

*Pretreatment/impregnation of bagass*

After the juice extraction, the bagass is further processed via route A. Optionally, part of the bagass may be used in steam or power production. The power or steam may be used in the ethanol production and may hence be brought back to the ethanol production line in the form of energy (not shown in Figure 3). For example, part of the bagass may be used in production of steam, which is subsequently used in the pretreatment or the distillation.

When the bagass is further processed it is transported via route A to a pretreatment unit 305. The pretreatment unit may be a vessel or a container.

An effective pretreatment is needed to render the cellulose of the bagass accessible to the enzymes in the subsequent hydrolysis step. In one example, the bagass is first impregnated before subsequent heat treatment. Impregnation refers to impregnating the cellulose biomass with an impregnation fluid. The impregnation fluid may be an acid solution, such as a mineral acid solution. The impregnation may be performed with acid solutions having different pH, such as a pH of 0.5-5.5, such as 1.5-2.3 or such as a pH of 0.5-2. The impregnation may also be performed with a gas, such as a SO2-gas, or with the combination of a gas and liquid.

Other mineral acids that may be used are hydrochloric acid, nitric acid, phosphoric acid, boric acid and hydrofluoric acid.

Subjecting the bagass to at least one impregnation fluid fluid may be performed by different techniques known to the skilled person.

In one example, the cellulosic biomass is impregnated in a cellulosic biomass/liquid ratio of about from 1:1 to 1:7 to provide an impregnated cellulosic biomass.

*Hydrolysis*

After pretreatment the impregnated bagass, is transported to the hydrolysis unit 306.

The acidic hydrolysis may be performed at a certain pH, a certain temperature and a certain pressure during a certain time. Acidic hydrolysis of pretreated cellulosic biomass is a well established technique, and it is within the capabilities of the skilled artisan to adjust the pH, temperature, pressure and time to achieve a satisfactory result. For example, the acidic hydrolysis may be performed at a temperature of 160-240 °C, at a pressure of 6-34 bar and during 1-60 min.

In one example, the impregnated bagass is treated with heat in two steps. For example, the pretreated bagass is further processed at 160-200 °C in a first step and at 200 °C or higher-, such as 200- 240 °C, in a second step.

Acid may be added to the bagass during the impregnation. Alternatively, or as a complement, acid is added during heat treatment.

The hydrolysate is neutralized before the fermentation. For example, the hydrolysate may be neutralized by means of an addition of NaOH or ammonia. Also, lime stone (CaOH) may be used, which is a cheap alternative.

*Fermentation*

The cane hydrolysate comprising the fermentable sugars is subjected to fermentation 308 in an aqueous liquid fermentation utilizing at least one fermenting agent, which can be yeast. The yeast may be wild type, mutant or recombinant *Saccaromyces cerevisae.* Suitably, the yeast is dissolved in sterilized water before its addition. Regarding the addition of yeast, it is mainly considered in the beginning of the process, since it is long living and also reproducing. If the yeast by any reason performs poorly and/or dies, new yeast has to be added.

The fermentation takes place at a temperature of 30 to 37°C and a certain pH. The fermentation is an exothermic reaction, and therefore cooling is mormally needed to keep the temperature within the described range.

The pH during the fermentation should preferably be in the range of 4.8 to 6, more preferably 5.3 to 5.7. It may be necessary to measure the pH throughout the fermentation and adjust the pH if needed, partly because acetic acid is formed/released during the process.After the extraction and optionally the clarification and evaporation, the juice C is mixed with the hydrolysed bagass A before or during the fermentation 308 via route 307.

During the merge of flows A and C, the proportions of the respective amounts are controlled such that the fermenting agent is stressed to an extent where an improved ethanol yield is obtained. Different proportions of the flows may be tested and the resulting ethanol yield measured. The proportions resulting in the best ethanol yield are selected, and subsequently, the flows are controlled to be merged in such a proportion.

A low sugar concentration in the fermentor promotes the xylose fermentation. Consequently, it may be beneficial to keep the sugar concentration low. The sugar concentration may be kept low by adding the bagass-derived material first and allowing the fermentation to proceed for a while without any sugar addition. Then, the sugar is added continuously during a period such that the glucose concentration in the SSF is 2-3 g/l. Finally, the fermenting is allowed to proceed for a while after the sugar addition is completed. In this configuration, the fermenting agent(s) employed is/are capable of fermenting pentoses.

*Distillation*

Regardless of which type of hydrolysis that has been used, after fermentation 211, 212, 308, the mash may be transported to a distillation apparatus 213, 309. Distillation is a preferred method for separating ethanol from the fermented hydrolysate due to the lower boiling point of ethanol compared to the other substances comprised in the fermented hydrolysate.

In the distillation apparatus, the mash is heated to the boiling point of ethanol, 78.3°C, which results in that the ethanol is leaving the apparatus in the form of gas. The ethanol gas is cooled down and the ethanol is obtained in the liquid state. At the same time, the remaining fluid and the solid residues (mainly lignin and/or degraded products of lignin) are drained off from the lower part of the plant (not shown in Figure 2 and Figure 3). If the solids should be prevented from accompanying into the distillation apparatus 213, 309, the mash can pass a separation unit of any kind, for example a laminated separator.

*Dehydration*

After distillation 213, 309 the mash may be dehydrated in a dehydration unit 214, 310 in order to increase the concentration of ethanol.

Example 1

The following experiment was performed in a laboratory with the intention to simulate an industrial method according to the present disclosure. Also control experiments (zero samples) were performed.

The raw material was composed of the grain wheat. The wheat straw was obtained from one place and the wheat starch was bought as flour, the product Kungsörnen® (Järna, Sweden), in a grocery store.

The wheat straw was disintegrated (chopped) by means of a hammer mill. The material was bolted and the pieces, which had a length of between 2 and 10 mm, were kept. The straw pieces were stored at room temperature until it was time for the processing.

The straw pieces were immersed in a 0.2% sulphuric acid solution by weight (20 gram of fluid per gram of straw). After an hour of rest at room temperature in the acid solution, the straw pieces were pressed to a dry content of 40 percentage of weight. The straw, in sets of 600 gram, with a stated dry content, was treated with water steam for 10 minutes at a temperature of 190°C and corresponding water steam pressure. The steam treatment equipment was composed of a reactor, which holds 10 liters, plus a flash tank which collected the treated material. The reactor was composed of a vertical cylinder with a ball valve in the top, where the material was introduced, and an additional valve in the bottom, which was controlled by a computer. When the predetermined retention time was reached, the valve below was opened and the material was flinged into the cyclone (flash tank). There were two steam channels into the reactor, one in the bottom and one in the top. The lower one was opened during a short time in the beginning of the treatment to achieve a fast heating of the material, while the upper was controlling the temperature and pressure during the entire treatment.

The untreated straw as well as the straw after described acid- and steam treatment was analyzed and the components are shown below in weight percentage of dry material.

Table 1

| Component | Straw | Treated straw |
|---|---|---|
| Glucan | 37.4 ± 0.14 | 47.7 ± 3.9 |
| Mannan | not detectable | 4.7 ± 0.3 |
| Xylan | 21.0 ± 0.2 | 3.0 ± 0.1 |
| Galactan | 1.6 ± 0.0 | 1.6 ± 0.1 |
| Arabinan | 3.2 ± 0.1 | 1.9 ± 0.1 |
| Acid soluble lignin | 3.2 | 2.1 |
| Acid insoluble lignin | 18.4 | 26.5 |
| Lignin, total | 21.6 | 28.6 |
| Lignin ashes | 1.1 | 0.0 |
| Total | 87.4 | 85.8 |

The saccharides were analyzed by means of acid hydrolysis and HPLC ("High Performance Liquid Chromatography") and the lignin was analyzed by means of absorption spectrophotometry.

The aggregated value is not 100 and this is due to the fact that the straw contains more chemical substances than those which were analyzed.

Initially, the wheat was hydrolyzed and this occurred in the following way. A first three hour-liquefaction step at a temperature of 85°C was performed. To the dry flour a water solution, comprising an enzyme, was added in such an amount that a slurry with a dry content of 35% was obtained. The enzyme was a thermo stable alpha-amylase in the form of Thermacyl SC and the added amount was 0.5 gram per kilogram of dry wheat flour. The pH-value was set to 5.5 by addition of 72% sulphuric acid solution by weight. Thereafter, the slurry was cooled down to 60°C and the starch was saccharified for five hours at this temperature. To the slurry an amyloglucosidase was added in the form of Spirizin Fuel, which was added in an amount of 0.5 milliliter per kilogram of dry wheat flour. Further, additional 72% sulphuric acid solution by weight was added leading to a decrease of the pH-value to 4.2. After this treatment, 60% starch by weight was saccharified.

The above described treatment was performed in a Rotavapor with a volume of 1 liter. The equipment was Büchi Rotavapor R-153 (Büchi Labortechnik AG, Flavil, Switzerland).

Thereafter, the two material flows were mixed and exposed to simultaneous saccharification and fermentation (SSF). Experiments were performed on both the individual material flows as well as mixtures of both material flows in different proportions. The amount of treated straw and/or saccharified starch added was all cases based on the original amount of solid material, i.e. material not dissolved in water. The content of such material was in all cases five percentage of weight. The experiments were performed with a total weight of 1.4 kilogram of slurry in a laboratory fermentor with a volume of 2 liters.

The treated wheat straw was sterilized in the fermentor for 20 minutes at a temperature of 121°C just before the experiments. In the experiments, a nutrient solution comprising 0.5 gram/liter $(NH_4)_2$ $HPO_4$ and 0.025 gram/liter $MgSO_4$ and 1 gram/liter yeast extract was added. Also the nutrient solution was sterilized. Since the treated straw and especially the cellulose in the same had not been saccharified, enzymes for converting cellulose to glucose were added. Two types of enzymes were added in the experiments where the straw was present; Cellulast 1.5 L in an amount of 20 FPU per gram of cellulose, and Novozyme 188 in an amount of 23 IU per gram of cellulose. The enzymes were not sterilized before the addition. Neither was the fermenting agent, i.e. the yeast, sterilized before its addition. However, the yeast was dissolved in cold water before the addition. The yeast was a strain of Saccharomyces cerevisiae (ordinary baking yeast from Jästbolaget, Rotebro, Sweden) and was added in an amount of 5 gram per liter. The experiments were performed for 72 hours at a temperature of 36.5°C and the pH-value in the slurry was 5.

The control of the pH-value was performed by addition of a 10% sodium hydroxide solution by weight during the experiments. The produced carbon dioxide was cooled and the condensed fluid, which contained ethanol and water, was brought back to the fermentor.

The ethanol yield which was obtained in the different experiments (after 72 hours of SSF), and also the measured level of a few inhibitors (in the beginning of the experiments) in these experiments are shown in Table 2 below.

EP 2 240 591 B2

Table 2

| Experiment | Amount of straw (%) | Amount of starch (%) | Fermentable sugars from straw (%) | Fermentable sugars from starch (%) | Ethanol yield (%) | Stress factor (%) | Acetic acid (g/l) | Furfural (g/l) | HMF (g/l) |
|---|---|---|---|---|---|---|---|---|---|
| 1 | 0 | 100 | 0 | 100 | 81.1 | 1 | 0 | 0 | 0 |
| 2 | 50 | 50 | 26 | 74 | 97.1 | 1.19 | 0.72 | 0.66 | 0.15 |
| 3 | 60 | 40 | 35 | 65 | 89.2 | 1.09 | 0.88 | 0.75 | 0.19 |
| 4 | 70 | 30 | 46 | 54 | 85.1 | 1.05 | 1.01 | 0.90 | 0.22 |
| 5 | 85 | 15 | 67 | 33 | 82.0 | 1.01 | 1.24 | 1.08 | 0.27 |
| 6 | 100 | 0 | 100 | 0 | 80.2 | 0.99 | 1.46 | 1.28 | 0.32 |

HMF is an abbreviation of hydroxymethylfurfural.

To highlight the effect of the addition of treated straw, the ethanol yield was calculated on the content of fermentable sugar into the fermentation. The ethanol yield is expresses in percentage of the theoretically possible amount of ethanol that can be obtained. The formula for the calculation is:

$$\text{Ethanol yield in \%} = \frac{\text{ethanol out (grams)}}{\text{sugar in (grams)} * 0.51} * 100$$

As shown in Table 2, the ethanol yield was 81.1 % when the conventional way of recovery of saccharides from a material, i.e. the one line concept, was used. When the two line concept was used and the material flows were mixed in the same proportion, i.e. 50% of treated straw and 50% of wheat flour starch, the ethanol yield raised to surprisingly high 97.1 %. Increased amount of treated straw, at the expense of the wheat flour starch, reduces the yield gradually, though the ethanol yield was not only reduced for wheat flour starch. If the two line concepts are compared with each other it is found that the ethanol yield for the lignocellulosic material, i.e. the pretreated straw, is barely noticeably below compared to the ethanol yield for the wheat flour starch. This can be indicative of that the lignocellulosic material flow has to be diluted because it is strongly inhibited. By diluting with a saccharide-containing material flow, an optimal effect of the inhibitors may be obtained, i.e. an increased ethanol yield.

As expected, the amount of the three analyzed inhibitors in the mash is reduced with the reduced addition of the treated straw, i.e. the lignocellulosic material. It should be noticed that there are many other substances which affect the yeast in addition to the analyzed inhibitors. However, the data of table 2 clearly indicates that one or a few of these substances (the analyzed and/or the other present) in a suitable amount provides a positive, here called stressful, effect on the yeast fungus. An additional reduction of the lignocellulosic material part (less than 50%), or more correct the part of the saccharide-containing solution/suspension emanating from the lignocellulosic material, may result in an additionally increased ethanol yield.

The theoretical proportion of fermentable sugars contributed by each starting material in the experiments was calculated. The flour was assumed to consist of 100 % starch, and 1 kg of starch was assumed to provide 1.11 kg glucose (after hydrolysis). 1 kg of straw was assumed to provide 0.4 kg of glucose. A wide range of proportions is shown in Table 2 to provide an increased ethanol yield, i.e. the range from 0.26:0.74 to 0.67:0.33 (sugars (straw):sugars (starch)).

Comparative example

Table 3 shows the concentrations of some constituents of bagass-derived products obtained after different types of steam pretreatment. The table shows that the inhibitors furfural, acetic acid and HMF are released during the steaming, and thus, that they form part of bagass-derived treatment products.

Table 3. Characteristics of the bagass-derived products obtained after steam pretreatment.

| Catalyst | Log ($R_o$) | pH | Concentration (g/l) | | | | | |
|---|---|---|---|---|---|---|---|---|
| | | | xylose | arabinose | glucose | HOAc | Furfural | HMF |
| None | 3.05 | 4.4 | 2.69 | 0.87 | 2.24 | 0.03 | 0.95 | 0.10 |
| | 3.35 | 4.2 | 3.99 | 0.73 | 0.76 | 0.15 | 0.62 | 0.09 |
| | 3.65 | 4.0 | 2.46 | 0.34 | 0.51 | 0.29 | 0.94 | 0.08 |
| | 4.09 | 3.7 | 8.83 | 0.98 | 0.95 | 0.62 | 0.95 | 0.09 |
| $SO_2$ | 3.05 | 1.8 | 30.50 | 2.73 | 3.87 | 2.64 | 0.68 | 0.02 |
| | 3.35[1] | 1.8 | 20.99 | 1.94 | 3.03 | 2.74 | 0.94 | 0.10 |
| | 3.35[2] | 1.7 | 17.61 | 1.53 | 3.04 | 2.58 | 0.98 | 0.10 |
| | 3.65 | 1.8 | 12.80 | 1.56 | 2.77 | 1.92 | 1.05 | 0.14 |
| | 4.09 | 1.7 | 16.43 | 1.52 | 3.54 | 2.79 | 1.03 | 0.15 |
| $H_2SO_4$ | 3.35 | 3.3 | 11.89 | 1.59 | 1.70 | 0.04 | 0.95 | 0.08 |
| | 3.65 | 3.6 | 5.72 | 0.82 | 0.89 | 0.34 | 0.94 | 0.12 |
| | 4.09 | 3.3 | 12.56 | 1.42 | 1.75 | 0.69 | 0.94 | 0.10 |

(1) 180°C/ 10 min. (2) 190°C/5 min. HOAc = acetic acid.

(The use of the severity factor *Log($R_o$)* simplifies the comparison of results from different pretreatment experiments. The temperature and the residence time are combined to form one expression:

$$Log(Ro) = Log\left( t \cdot \exp\left( \frac{(T - T_{ref})}{14.75} \right) \right)$$

where $t$ is the residence time in minutes, $T$ is the reaction temperature in °C and $T_{ref}$ is the reference temperature, which is set to 100°C.)

## Claims

1. A method of improving the ethanol yield in production of an ethanol-containing product from a lignocellulosic biomass comprising straw and a starch-rich biomass comprising grain, comprising:

a first treatment, involving hydrolysis, of said lignocellulosic biomass in one or more steps to obtain lignocellulose-derived treatment products including fermentable sugars and substances capable of stressing the fermenting agent comprising furfural, acetic acid and HMF; and

a second treatment, involving hydrolysis, of said starch-rich biomass in one or more steps to obtain starch-derived fermentable sugars;

fermentation, using a fermenting agent, of a mixture comprising at least part of said lignocellulose-derived treatment products and at least part of said fermentable sugars derived from said starch-rich material to obtain said ethanol-containing product,

wherein an amount of lignocellulose-derived material and an amount of material derived from the starch-rich biomass are mixed in the fermentation or earlier such that said at least part of said lignocellulose-derived treatment products and said at least part of said starch-derived fermentable sugars are present in the mixture, and said amounts are controlled such that said mixture comprises furfural in a concentration of 0.2 to 0.9 g/l, acetic acid in a concentration of 0.35 to 8 g/l and HMF in a concentration of 0.015 to 0.75 g/l and said fermenting agent is subjected to stress by lignocellulose-derived treatment products to the extent that said ethanol yield is improved,

wherein the hydrolysis of the first treatment comprises enzymatic hydrolysis.

2. Method according to claims 1, wherein said first treatment comprises pretreatment and said mixing takes place after said pretreatment of said lignocellulosic biomass.

## Patentansprüche

1. Verfahren zur Verbesserung der Ethanolausbeute bei der Herstellung eines ethanolhaltigen Produkts aus einer Lignocellulose-Biomasse umfassend Stroh und einer stärkereichen Biomasse umfassend Korn umfassend:

eine Hydrolyse umfassende erste Behandlung der Lignocellulose-Biomasse in einem oder mehreren Schritten, um von Lignocellulose abgeleitete Behandlungsprodukte zu erhalten, einschließlich fermentierbarer Zucker und Substanzen, die das Fermentationsmittel beanspruchen könnten, umfassend Furfural, Essigsäure und HMF; und

eine Hydrolyse umfassende zweite Behandlung der stärkereichen Biomasse in einem oder mehreren Schritten, um von Stärke abgeleitete fermentierbare Zucker zu erhalten;

Fermentation einer Mischung, die mindestens einen Teil der von Lignocellulose abgeleiteten Behandlungsprodukte und mindestens einen Teil der von dem stärkereichen Material abgeleiteten fermentierbaren Zucker umfasst, unter Verwendung eines Fermentationsmittels, um das ethanolhaltige Produkt zu erhalten,

wobei eine Menge an von Lignocellulose abgeleitetem Material und eine Menge an Material, das von der stärkereichen Biomasse abgeleitet ist, bei der Fermentation oder früher gemischt werden, derart, dass der mindestens eine Teil der von Lignocellulose abgeleiteten Behandlungsprodukte und der mindestens eine Teil der von Stärke abgeleiteten fermentierbaren Zucker in der Mischung vorhanden sind,

und die Mengen derart gesteuert werden, dass die Mischung Furfural in einer Konzentration von 0,2 bis 0,9 g/l, Essigsäure in einer Konzentration von 0,35 bis 8 g/l und HMF in einer Konzentration von 0,015 bis 0,75 g/l umfasst und das Fermentationsmittel einer Beanspruchung durch von Lignocellulose abgeleitete Behandlungsprodukte in dem Ausmaß ausgesetzt wird, dass die Ethanolausbeute verbessert wird,
wobei die Hydrolyse der ersten Behandlung eine enzymatische Hydrolyse umfasst.

**2.** Verfahren nach Anspruch 1, wobei die erste Behandlung eine Vorbehandlung umfasst und das Mischen nach der Vorbehandlung der Lignocellulose-Biomasse stattfindet.


**Revendications**

**1.** Procédé d'amélioration du rendement en éthanol dans la production d'un produit contenant de l'éthanol à partir d'une biomasse lignocellulosique comprenant de la paille et d'une biomasse riche en amidon comprenant des grains, comprenant :

un premier traitement, impliquant une hydrolyse, de ladite biomasse lignocellulosique en une ou plusieurs étapes afin d'obtenir des produits de traitement dérivés de lignocellulose comportant des sucres fermentescibles et des substances capables de stresser l'agent de fermentation comprenant du furfural, de l'acide acétique et du HMF ; et
un second traitement, impliquant une hydrolyse, de ladite biomasse riche en amidon en une ou plusieurs étapes pour obtenir des sucres fermentescibles dérivés d'amidon ;
la fermentation, en utilisant un agent de fermentation, d'un mélange comprenant au moins une partie desdits produits de traitement dérivés de lignocellulose et au moins une partie desdits sucres fermentescibles dérivés dudit matériau riche en amidon pour obtenir ledit produit contenant de l'éthanol,
dans lequel une quantité de matériau dérivé de lignocellulose et une quantité de matériau dérivé de la biomasse riche en amidon sont mélangées au cours de la fermentation ou plus tôt de telle sorte que ladite au moins une partie desdits produits de traitement dérivés de lignocellulose et ladite au moins une partie desdits sucres fermentescibles dérivés d'amidon sont présentes dans le mélange,
et lesdites quantités sont régulées de telle sorte que ledit mélange comprend du furfural en une concentration de 0,2 à 0,9 g/l, de l'acide acétique en une concentration de 0,35 à 8 g/l et du HMF en une concentration de 0,015 à 0,75 g/l et ledit agent de fermentation est soumis à un stress par les produits de traitement dérivés de lignocellulose à un point tel que ledit rendement en éthanol est amélioré,
dans lequel l'hydrolyse du premier traitement comprend une hydrolyse enzymatique.

**2.** Procédé selon la revendication 1, dans lequel ledit premier traitement comprend un prétraitement et ledit mélange a lieu après ledit prétraitement de ladite biomasse lignocellulosique.

**FIGURE 1**

FIGURE 2

FIGURE 3

**EP 2 240 591 B2**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *App. Biochem. Biotech.,* 2002, vol. 98-100, 5-21
- **SÖDERSTRÖM.** *Biomass Bioenergy,* 2003, vol. 24, 475-486
- **SÖDERSTRÖM.** *App. Biochem. Biotech.,* 2005, vol. 121-124, 1055-1067